(19) Europäisches Patentamt
European Patent Office
Office européen des brevets

(11) EP 1 104 674 A1

(12) EUROPÄISCHE PATENTANMELDUNG

(43) Veröffentlichungstag: **06.06.2001 Patentblatt 2001/23**

(21) Anmeldenummer: **99122346.2**

(22) Anmeldetag: **10.11.1999**

(51) Int Cl.[7]: **A61K 31/655**, A61P 7/04, A61P 7/06, A61P 43/00, C09B 43/00

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Curacyte AG**
**80339 München (DE)**

(72) Erfinder:
- **Voss, Edgar, Dr.**
**68519 Viernhem (DE)**
- **Brandt, Michael, Dr.**
**82393 Iffeldorf (DE)**

(74) Vertreter: **Böhm, Brigitte, Dipl.-Chem. Dr. et al**
**Weickmann & Weickmann**
**Patentanwälte**
**Postfach 86 08 20**
**81635 München (DE)**

# (54) O,o'-Dihydroxyazofarbstoffe als Bestandteile von Arzneimitteln mit TPO-Agonistischer oder -Synergetischer Wirkung

(57) Gegenstand der Erfindung sind o,o'-Dihydroxyazoverbindungen, die eine synergistische und/oder agonistische Wirkung auf den TPO-Rezeptor (mpl-Rezeptor) besitzen und ihre Anwendung als Arzneimittel zur Behandlung von Thrombopenien und Anämien sowie zur Mobilisierung oder Expansion von Stamm- oder Progenitorzellen, zur Stimulation der Megakaryozyten-, Plättchen- oder Erythrozytenbildung.

EP 1 104 674 A1

## Beschreibung

Gegenstand der vorliegenden Erfindung sind o,o'-Dihydroxyazoverbindungen, die eine agonistische und/oder synergistische Wirkung auf den TPO-Rezeptor besitzen und diese enthaltende Arzneimittel.

Die Erfindung betrifft Dihydroxyazofarbstoffe der allgemeinen Formel **I**

$$X—N=N—Y \qquad (I)$$

**X =** 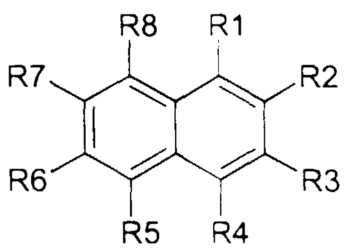 **Y =** 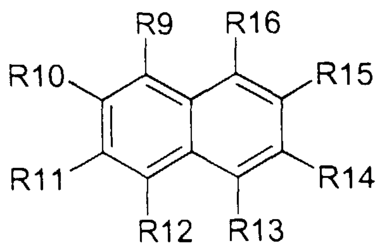

oder

**Y =** 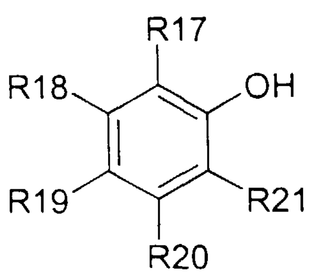

in welcher

- $R^1$ Hydroxy oder eine Bindung zur Azogruppe bedeutet,
- $R^2$ Hydroxy bzw. eine Bindung zur Azogruppe bedeutet wenn $R^1$ einer Hydroxygruppe entspricht.
- $R^3$ Wasserstoff. Halogen, $C_1$-$C_4$-Alkyl, $C_1$-$C_4$-Alkoxy oder ein Carbonsäureamid welches am Stickstoff durch eine Phenylgruppe, die gegebenenfalls substituiert sein.
- $R^4$ Wasserstoff, Halogen oder eine Sulfonsäuregruppe,
- $R^5$ Wasserstoff, Halogen, $C_1$-$C_4$-Alkyl, $C_1$-$C_4$-Alkoxy oder eine Sulfonsäuregruppe,
- $R^6$,$R^7$ Wasserstoff, Halogen. $C_1$-$C_4$-Alkyl, $C_1$-$C_4$-Alkoxy,
- $R^8$ Wasserstoff, Halogen, $C_1$-$C_4$-Alkyl, $C_1$-$C_4$-Alkoxy oder Acetamido.
- $R^9$ Hydroxy oder eine Bindung zur Azogruppe bedeutet.
- $R^{10}$ Hydroxy bzw. eine Bindung zur Azogruppe bedeutet wenn $R^9$ einer Hydroxygruppe entspricht.
- $R^{11}$ Wasserstoff, Halogen, Methoxy oder eine Carboxylgruppe.
- $R^{12}$ Wasserstoff, Halogen. $C_1$-$C_4$-Alkoxy oder eine Sulfonsäuregruppe,
- $R^{13}$ Wasserstoff, Halogen, $C_1$-$C_4$-Alkyl, $C_1$-$C_4$-Alkoxy oder eine Sulfonsäuregruppe,
- $R^{14}$ Wasserstoff, Halogen, $C_1$-$C_4$-Alkyl, $C_1$-$C_4$-Alkoxy oder Nitro,
- $R^{15}$ Wasserstoff, Halogen, $C_1$-$C_4$-Alkyl, $C_1$-$C_4$-Alkoxy oder eine Sulfonsäuregruppe,
- $R^{16}$ Wasserstoff oder Halogen,
- $R^{17}$ Wasserstoff, Halogen oder eine Sulfonsäuregruppe,
- $R^{18}$ Wasserstoff, Halogen, Hydroxy oder eine Sulfonsäuregruppe, wobei $R^{18}$ auch Trifluormethyl bedeuten kann wenn R' gleichzeitig eine Hydroxygruppe bedeutet,
- $R^{19}$ Wasserstoff, Halogen. Methyl, Methoxy oder eine Sulfonsäuregruppe,
- $R^{20}$ Wasserstoff, Halogen oder $C_1$-$C_4$-Alkyl und

$R^{21}$ eine Bindung zur Azogruppe bedeutet.

$C_1$-$C_4$ bedeutet dabei Methyl, Ethyl, Propyl, Butyl, Isopropyl, Isobutyl oder tert-Butyl.

Gegenstand der Erfindung sind auch Tautomere, die E-, Z-Isomeren. die physiologisch verträglichen Salze und Prodrugs der Verbindungen der allgemeinen Formel **I**. Beispiele für physiologisch verträgliche Salze sind dabei Alkalimetall-, Erdalkalimetall-, Ammonium- und Alkylammoniumsalze, wie das Natrium-, Kalium-, Calcium- oder Tetramethyl-ammoniumsalz.

Bevorzugt sind Verbindungen der allgemeinen Formel **I** in denen $C_1$-$C_4$-Alkyl eine Methylgruppe und $C_1$-$C_4$-Alkoxy eine Methoxygruppe bedeutet.

o,o'-Dihydroxyazoverbindungen vom Typ der allgemeinen Formel **I** sind seit langem bekannt aus zahlreichen Veröffentlichungen und Patenten (z.B. DE 177925, DE 188645, DE 403552) und wurden in vielfältiger Art und Weise als Farbstoffe angewendet.

Neu sind Verbindungen der allgemeinen Formel **I** in denen $R^{18}$ eine Trifluormethylgruppe und $R^1$ gleichzeitig eine Hydroxygruppe bedeutet, diese Verbindungen sind ebenfalls bevorzugt, besonders 5-Hydroxy-6-(2-hydroxy-4-trifluormethyl-phenylazo)-naphthalin-1-sulfonsäure,sowie deren E-, Z-Isomere, Tautomere, Salze und Ester.

Überraschenderweise wurde nun gefunden, daß Verbindungen der allgemeinen Formel **I** eine bisher unbekannte TPO-agonistische und synergistische Wirkung zeigen. Sie eignen sich daher für die Behandlung von Krankheiten bei denen auch Thrombopoetin oder andere Proteine/Peptide, die an den mpl-Rezeptor (Thrombopoetin-Rezeptor) binden, als Therapeutikum eingesetzt werden. Insbesondere eignen sie sich zur Behandlung hämatopoietischer Störungen, beispielsweise bei der Therapie von Thrombopenien und Anämien, z.B. nach Chemo- oder Strahlentherapie bzw. Knochenmarkstransplantationen sowie zur Mobilisierung von Stamm- und Progenitorzellen.

Sie können auch für die *in vivo* und *in vitro* Expansion von Stammzellen zur Regeneration des hämatopoietischen Systems benutzt werden, und um modifizierte Stammzellen für Gentherapieanwendungen zur Verfügung zu stellen.

Im folgenden wird nur der Begriff TPO für alle, an mpl bindende Proteine/Peptide verwendet.

Unter den verschiedenartigen Zellen des Blutes, die bei einer Lebensdauer von nur wenigen Stunden bis zu 20 Tagen ständig neu gebildet werden müssen, stellen die von Progenitorzellen gebildeten Megakaryozyten eine wichtige Gruppe dar. Das Wachstum der Megakaryozyten und ihre Entwicklung wird durch hämatopoietische Wachstumsfaktoren reguliert. So bewirken diese einerseits die Expansion der Megakaryozytenprogenitoren (Megakaryopoiese), andererseits induzieren sie die Megakaryozytenreifung bis zur Thrombozytenbildung (Thrombopoiese). Thrombozyten, auch Blutplättchen genannt, sind kleine Zellen, die zur Blutgerinnung beitragen und durch ihre Fähigkeiten zur Aggregation Wunden verschließen. Megakaryozyten entlassen nach der Fragmentierung des Zytoplasmas Blutplättchen in den vaskulären Raum. Bei einem gesunden Mensch entstehen 3-10 Milliarden Thrombozyten pro Stunde aus den blutbildenden Zellen des Knochenmarks.

Durch die Chemo- oder Strahlentherapie bei der Krebsbehandlung, verschiedene infektiöse Erkrankungen, Leukämie oder aplastische Anämie kann eine lebensbedrohliche Schädigung der Blutzellen ausgelöst werden. Auch nach einer Knochenmarkstransplantation wird die Neusynthese großer Mengen hämatopoietischer Zellen nötig, in seltenen Fällen liegen auch angeborene Defekte als Ursache für eine verminderte Blutplättchenzahl vor.

Allein in den USA unterziehen sich jährlich über 250.000 Patienten einer Chemotherapie wovon mindestens ein Drittel an Thrombozytopenie erkrankt und daher etwa 10 Millionen Thrombozytentransfusionen erforderlich sind. Hierzu werden jedoch sehr viele Blutkonserven benötigt, außerdem treten Probleme wie Alloimmunisierung, mögliche Übertragung viraler und bakterieller Infektionen sowie kongestives Herzversagen auf.

Der für die humorale Regulation des Megakaryozytenwachstums und die Plättchenbildung verantwortliche Faktor, das TPO (Thrombopoetin) wurde erst 1994 von verschiedenen Gruppen isoliert [1-3]. Der physiologische Bindungsort für TPO ist der mpl-Rezeptor, welcher beispielsweise auf CD34⊕Zellen, Megakaryozyten und Blättchen vorhanden ist [4].

Neben seiner Wirkung auf die Megakaryopoiese stimuliert TPO auch die Erythropoiese [5] und erhöht deshalb auch die Bildung von Erythrozyten in myelosupprimierten, bestrahlten Mäusen, die mit einem Chemotherapeutikum behandelt wurden, außerdem konnte eine Erhöhung der Neutrophilen erreicht werden [5]. Ebenso wie in Tiermodellen bewirkt TPO auch in Tumorpatienten mit Thrombopenie eine Erhöhung der Blutplättchen [6,7] und zeigt gute Verträglichkeit (WO-A-96/15758; WO-A-97/16535).

Durch die Anwendung von TPO allein oder im Zusammenwirken mit EPO oder G-CSF. die stimulierende Faktoren für Erythrozyten bzw. Granulozytenbildung sind, sollte es daher möglich sein. höhere und häufigere Dosen bei der Strahlen- bzw. Chemotherapie zu erreichen und somit eine effektivere Krebstherapie zu ermöglichen.

Eine Behandlung mit dem Humanprotein TPO hat jedoch eine ganze Reihe von Nachteilen:

Als rekombinantes Protein ist es außerordentlich teuer, wegen der fehlenden oralen Bioverfügbarkeit muß es parenteral verabreicht werden und durch Proteasen kann es rasch zu unwirksamen Fragmenten abgebaut werden. WO-A-96/40750 beschreibt Peptide mit TPO-agonistischer Aktivität, hier existiert jedoch das gleiche Problem der mangelnden oralen Bioverfügbarkeit und Protease-Sensitivität, so daß diese Substanzen durch Injektion oder Infusion verabreicht

werden müssen.
Überraschenderweise zeigen niedermolekulare Verbindungen der allgemeinen Formel **I** eine bisher unbekannte TPO-agonistische und synergistische Wirkung, sie sind daher wertvolle Arzneimittel.
Verbindungen der allgemeinen Formel **I** können in flüssiger oder fester Form oral oder parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Buffer, Ethanol, Dimethylsulfoxid, Komplexbildner (wie Ethylendiamintetraessigsäure), hochmolekulare Polymere (wie flüssiges Polyethylenoxid) zur Viskositätsregelung oder Polyethylenderivate von Sorbitanhydriden. Feste Drägerstoffe sind z.B. Stärke, Lactose, Mannit, Methylcellulose, Talkum. hochdisperse Kieselsäure, höhermolekulare Polymere (wie Polyethylenglycole). Für die orale Applikation können gewünschtenfalls zusätzlich Geschmacks- und Süßstoffe enthalten sein.

Die verabreichte Dosis hängt vom Alter, dem beim Patienten vorhandenen TPO-Spiegel, dem Ausmaß der Erkrankung, der Art der gleichzeitig durchgeführten Behandlungen und der Art der gewünschten Wirkung ab. Üblicherweise beträgt die tägliche Dosis der aktiven Verbindung 0.01 bis 5 mg/kg.

Azoverbindungen der allgemeinen Formel **I** können auf bekannte Weise durch Umsetzung eines entsprechenden Diazoniumsalzes mit einem Phenol oder Naphthol (Beispiel 4) hergestellt werden (DE 177925, DE 188645, DE 403552, **Organic Syntheses** Coll. Vol. **II,** 35(1943)).

Verbindungen der allgemeinen Formel **I** in denen $R^{18}$ Trifluormethyl und R' gleichzeitig eine

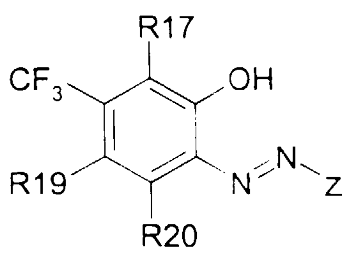

(**II**)

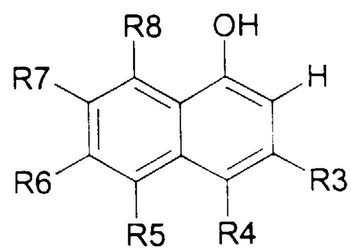

(**III**)

Hydroxygruppe bedeutet werden dargestellt durch Umsetzung eines Diazoniumsalzes **II** mit einem Naphthol der Formel **III** wobei Z ein Anion wie Chlorid, Bromid, Sulfat, Hydrogensulfat, Acetat, Hydroxid oder Sulfonat (in diesem Fall auch intramolekular) bedeutet und $R^3$, $R^4$, $R^5$, $R^6$, $R^7$, $R^8$, $R^{17}$, $R^{19}$und $R^{20}$ die bei Formel **I** angegebene Bedeutung besitzen (Beispiel 20).

<u>Ausführungsbeispiele:</u>

<u>Ausführungsbeispiel 1 (Beispiel 4 in Tabelle 1):</u>

<u>5-Hydroxy-6-(2-hydroxynaphthalin-1-ylazo)-naphthalin-1-sulfonsäure **(4)**</u>

680 mg ( 2.50 mmol ) 2-Diazo-1-naphthol-5-sulfonsäurenatriumsalz ( SHOWA KAKO Corporation / Japan) werden in 5.0 ml 2 molarer Salzsäure gelöst. Man rührt diese Lösung in eine Lösung von 360 mg 2-Naphthol in 6.0 ml 5 molarer Natronlauge ein. Es erfolgt ein Temperaturanstieg auf ca. 35 °C. Anschließend läßt man 20 Stunden bei Raumtemperatur nachrühren. Danach liegt eine tiefviolette Lösung vor. Nun wird unter Eiskühlung bei 10 - 15 °C 6 molare Salzsäure zugegeben bis ein pH von 4.0 erreicht ist. Dabei scheidet sich der Farbstoff als rotes Rohprodukt ab. Es wird abgesaugt und mit kaltem, destilliertem Wasser nachgewaschen und das Rohprodukt bei 40 °C im Vakuum getrocknet. Zur Reinigung chromatographiert man das Rohmaterial an Kieselgel (Laufmittel: Essigester/Methanol). Nach .Abziehen des Lösungsmittels am Rotationsverdampfer erhält man 0.2 g (21 %) 7 als rote Kristalle vom Schmp. > 300°C. MS(ESI-neg.): m/e = 393.

Ausführungsbeispiel 2 (Beispiel 20 in Tabelle 2):

5,Hydroxy-6-(2-hydroxy-4-trifluormethyl-phenylazo)naphthalin-1-sulfonsäure (**20**):

3-Trifluormethyl-6-nitrophenol (**21**)

24.10 g ( 18.1 ml, 150 mmol ) 3-Trifluormethylphenol werden in 25.0 ml 100 %iger Essigsäure gelöst. Zu dieser Lösung tropft man unter kräftigem Rühren und Kühlen bei -15 bis -10 °C, innerhalb von 45 Minuten, 16.1 g 65 %ige ( 10.4 ml, 150 mmol ) Salpetersäure zu. Nach beendigter Zugabe der Salpetersäure wird noch 1 Stunde bei -15 bis -10 °C nachgerührt. Anschließend entfernt man das Kältebad und läßt das Reaktionsgemisch auf Raumtemperatur kommen. Das Reaktionsgemisch wird auf 100 ml Eis gegossen, worauf sich ein Öl abscheidet. Das Öl wird in 60 ml Ether aufgenommen und die wässrige Phase noch dreimal mit je 20 ml Ether extrahiert. Die vereinigten Ether-Extrakte werden zweimal mit je 15 ml Wasser gewaschen und darauf über Natriumsulfat getrocknet. Nach Abfiltrieren des Trockenmittels wird der Ether am Rotationsverdampfer bei maximal 20 °C Badtemperatur abgezogen. Es bleibt ein gelbes Öl zurück, welches aus einem Gemisch von 3-Trifluormethyl-6-nitrophenol und 3-Trifluormethyl-4-nitrophenol besteht. Zur Trennung der Isomeren chromatographiert man an Kieselgel mit Heptan/Essigester 2 : 1 als Elutionsmittel. Neben 12.00 g (39%) 3-Trifluormethyl-4-nitrophenol erhält man dabei 6.20 g (20%) 3-Trifluormethyl-6-nitrophenol **9** als gelbes Öl. $^1$H-NMR($CDCl_3$): $\delta$ = 7.22(dd, 1H, 4-H), 7.46(d, 1H, 2-H), 8.23(dd, 1H, 5-H), 11.9(s, 1H, OH); $^{19}$F-NMR ($CDCl_3$): $\delta$ = -59.8 ppm.

3-Trifluormethyl-6-aminophenol (**22**)

2.60 g ( 12.5 mmol ) 3-Trifluormethyl-6-nitrophenol **21** werden in 50.0 ml Ethanol gelöst. Zu dieser Lösung gibt man 400 mg 10% Palladium auf Aktivkohle hinzu und läßt bis zur vollständigen Wasserstoffaufnahme hydrieren ( Theoretische Wasserstoff-Aufnahme = 844 ml ; praktische Wasserstoff-Aufnahme = 850 ml nach 2 Stunden ). Anschließend wird vom Katalysator abgesaugt. Das Filtrat versetzt man mit 7.0 ml 2 molarer Salzsäure und destilliert dann am Rotationsverdampfer zur Trockene ab. Der Rückstand wird mit Isohexan verrieben, abgesaugt und darauf im Vakuum bei 40 °C getrocknet. Man erhält 2.50 g ( 94 %) **22** als weißes, amorphes Hydrochlond. $^1$H-NMR($CDCl_3$): $\delta$ = 7.19 (dd, 1H, 4-H), 7.28 (d, 1H, 2-H), 7.43 (dd, 1-H, 5-H).

5-Hydroxy-6-(2-hydroxy-4-trifluormethyl-phenylazo)naphthalin-1-sulfonsäure (**20**)

4.20 g ( 20.0 mmol ) 2-Hydroxy-4-trifluormethyl-anilinhydrochlorid **22** werden in 6.0 ml konzentrierter Salzsäure suspendiert. Zu dieser Suspension tropft man unter Rühren und Kühlen bei 0 - 5 °C eine Lösung von 1.40 g ( 20.0 mmol ) Natriumnitrit in 6.0 ml destilliertem Wasser hinzu. Nach vollständiger Zugabe der Natriumnitritlösung liegt eine gelbe Lösung vor. Es wird 15 Minuten bei 0 - 5 °C nachgerührt. Die auf diese Weise frisch bereitete Diazoniumsalzlösung wird bei Raumtemperatur in eine Lösung von 5.00 g ( 20.0 mmol) 5-Hydroxynaphthalin-1-sulfonsäure-mono-natriumsalz in 25.0 ml 2 molarer Natronlauge eingerührt, wobei die Temperatur auf ca. 30 - 35 °C ansteigt. Man läßt nun 48 Stunden bei Raumtemperatur nachrühren. In dieser Zeit scheidet sich die 5-Hydroxy-6-(2-hydroxy-4-trifluormethyl-phenylazo)naphthalin-1-sulfonsäure als mono-Natriumsalz ab. Es wird nun abgesaugt und mit wenig kaltem, destilliertem Wasser nachgewaschen, worauf das so erhaltene Rohprodukt im Vakuum bei 40 °C getrocknet wird. Die Aufreinigung des Rohproduktes erfolgte durch Flashchromatographie (Laufmittel: Essigester/Methanol 2:1) an Kieselgel. Nach Abdestillieren des Lösungsmittels und Verreiben des Rückstandes mit Essigester erhält man das dunkelrote mono-Natriumsalz von 20 mit Schmp. > 300°C, MS(neg. ESI-Spektrum): m/z = 411.

In analoger Weise werden die Ausführungsbeispiele **1-3, 5-12** (Tabelle 1) sowie **13-19** (Tabelle 2) erhalten.

## Tabelle 1

X—N=N—Y

X = 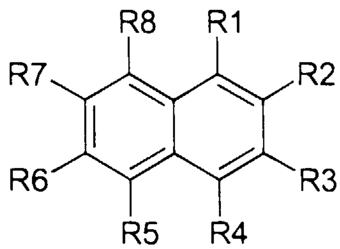

Y = 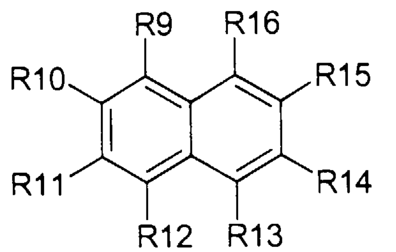

| Beisp | R1 | R2 | R3 | R4 | R5 | R6 | R7 | R8 | R9 | R10 | R11 | R12 | R13 | R14 | R15 | R16 |
|---|---|---|---|---|---|---|---|---|---|---|---|---|---|---|---|---|
| 1 | OH | A | H | H | SO3H | H | H | H | OH | A | H | OCH3 | H | H | H | H |
| 2 | OH | A | H | SO3H | H | H | H | H | OH | A | H | OCH3 | H | H | H | H |
| 3 | OH | A | H | H | SO3H | H | H | H | OH | A | H | H | SO3H | H | H | H |
| 4 | OH | A | H | H | SO3H | H | H | H | A | OH | H | H | H | H | H | H |
| 5 | OH | A | H | H | SO3H | H | H | H | A | OH | H | H | H | H | SO3H | H |
| 6 | OH | A | H | H | SO3H | H | H | H | A | OH | COOH | H | H | H | H | H |
| 7 | OH | A | H | SO3H | H | H | H | H | A | OH | H | H | H | H | H | H |
| 8 | OH | A | H | SO3H | H | H | H | H | A | OH | H | H | H | H | SO3H | H |
| 9 | OH | A | H | H | H | H | H | H | A | OH | H | SO3H | H | NO2 | H | H |
| 10 | OH | A | H | H | H | H | H | H | A | OH | H | SO3H | H | H | H | H |
| 11 | A | OH | H | H | H | H | H | H | A | OH | H | SO3H | H | H | H | H |
| 12 | A | OH | H | H | H | H | H | H | A | OH | H | SO3H | H | NO2 | H | H |

**A = Bindung zur Azogruppe**

## Tabelle 2

X = 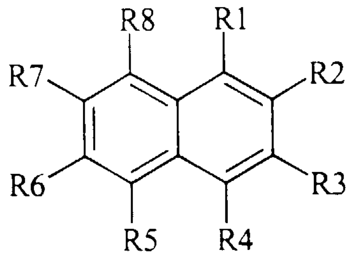

Y = 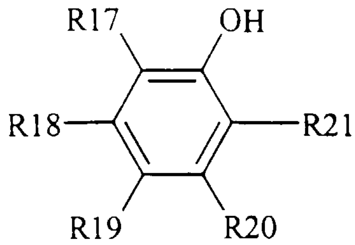

| Beispiel | R1 | R2 | R3 | R4 | R5 | R6 | R7 | R8 | R17 | R18 | R19 | R20 | R21 |
|---|---|---|---|---|---|---|---|---|---|---|---|---|---|
| 13 | A | OH | H | SO3H | H | H | H | H | H | H | CH3 | H | A |
| 14 | A | OH | CONH(2,4-Di-CH3Ph) | H | H | H | H | H | H | H | H | H | A |
| 15 | A | OH | CONH(2,4-Di-CH3Ph) | H | H | H | H | H | H | H | SO3H | H | A |
| 16 | A | OH | H | H | H | H | H | H | H | H | SO3H | H | A |
| 17 | A | OH | H | H | H | H | H | H | H | H | Cl | H | A |
| 18 | A | OH | H | H | H | H | H | NHCOCH3 | SO3H | H | Cl | H | A |
| 19 | OH | A | H | H | SO3H | H | H | NHCOCH3 | SO3H | H | Cl | H | A |
| 20 | OH | A | H | H | SO3H | H | H | H | H | CF3 | H | H | A |

**A = Bindung zur Azogruppe**

Pharmakologische Untersuchungen

Die Bioaktivität der erfindungsgemäßen Verbindungen kann mittels eines TPO-abhängigen Zellproliferationstests gemessen werden. Die Substanzen dürfen keine Wirkung auf die nicht transfektierten parenteralen BaF3 Zellen ausüben. Murine IL-3-abhängig wachsende BaF3 Zellen [8] wurden mit dem humanen mpl-Rezeptor transfektiert. In Abwesenheit von IL-3 ist die Proliferation und das Überleben dieser Zellen abhängig von TPO. Die parentale, untransfektierte Zell-Linie reagiert nicht auf humanes TPO, proliferiert aber in Gegenwart von IL-3. Die Bestimmung der Zellproliferation erfolgt nach literaturbekannten Verfahren (WO 96/40750). Die chemischen Substanz-Bibliotheken wurden in Bioassays mit den beiden obigen Zell-Linien gescreent. Die Zellen wurden in Gegenwart von IL-3 (BaF3 parental) bzw. TPO (BaF3-mpl-Rezeptor tragend = BaF3/mpl) in RPMI 1640 Medium in Gegenwart von 10 % FCS (fetales Kälberserum) gezüchtet. Für den Test wurden die Zellen zweimal in IL-3 bzw. TPO freiem Medium gewaschen und im Medium, das kein TPO bzw. IL-3 enthielt, resuspendiert. Diese Zellsuspension wurde dann zur Vertiefung einer 96 Tüpfelplatte (Costar), die TPO oder IL-3 und/oder Verbindung enthielt, in einer Menge von $10^4$ Zellen/Vertiefung zugegeben. Die Zellen wurden dann für 48 bis 72 h bei 37°C in einem $CO_2$ Inkubator inkubiert. Die proliferative Aktivität wurde bestimmt durch die Zugabe von WST (WST: Zell-Proliferations-Reagens; BM-Katalog-Nr. 1644807 "Tetrazolium-Salz").WST wird von proliferierenden Zellen zu Formazan umgewandelt und diese Umwandlung, als Maß für die Proliferation wird mittels OD (OD: optical density) bei 570 nm in einem ELISA Platten Meßgerät bestimmt.

Zur Ermittlung der halbmaximalen Stimulation wurde vom maximal erzielten Signal der Background (Zellen ohne Substanz) abgezogen und dieser Wert durch 2 geteilt. Dieser Wert plus dem Background-Wert wurden dann zur Bestimmung der $EC_{50}$ (halbmaximale excitatorv concentration; Substratkonzentration, bei der die Substanz ihre halbmaximale Wirksamkeit im BaF3-mpl Rezeptor Proliferationstest ausübt) herangezogen. In Tabelle 3 sind die $EC_{50}$-Werte bespielhaft für drei untersuchte Verbindungen dargestellt.

Die untersuchten Verbindungen stimulieren die Proliferation von mit mpl-Rezepror transfektierten BaF3 Zellen in einer Dosis abhängigen Weise. Die Proliferation von parenteralen Zell-Linien wird nicht stimuliert. Auch in Abwesenheit von TPO stimulieren die Verbindungen die Proliferation der BaF3/mpl-Zellen in wochenlanger Kultur.

Tabelle 3

| Beispiel Nr. | $EC_{50}$(µg/ml) |
|---|---|
| 9 | 0.6 |
| 12 | 0.7 |
| 13 | 2.0 |
| 15 | 2.2 |
| 20 | 0.03 |

[1] F. J. de Sauvage et al., *Nature* **1994**, *369*, 533-538
[2] Si Lok et al., *Nature* **1994**, *369*, 565-568
[3] T. D. Bartley et al., *Cell* **1994**, *77*, 1117-1124
[4] N. Methia et al., *Blood* **1993**, *82*, 1395-1401
[5] A. Grossmannet et al.. *Exp. Hematol.* **1996**, 24, 1238-1246
[6] R. Basser et al., *Blood* **1997**, *89*, 3118-3128
[7] M. Fanucchi et al., *New Engl. J. Med.* **1997,** 336, 404-409
[8] R. Palacios et al., *Cell* **1985**, *41*, 727-734

## Patentansprüche

**1.** Verwendung von Verbindungen der allgemeinen Formel **I**

$$X—N=N—Y \qquad (I)$$

X = 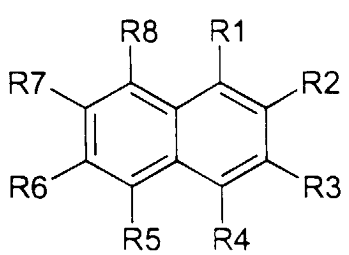

Y = 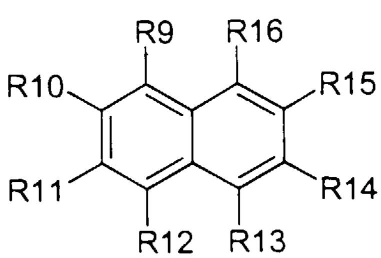

oder

Y = 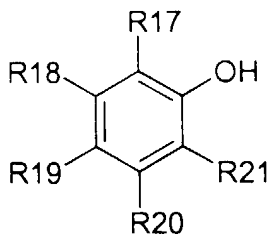

in welcher

$R^1$ Hydroxy oder eine Bindung zur Azogruppe bedeutet,
$R^2$ Hydroxy bzw. eine Bindung zur Azogruppe bedeutet wenn $R^1$ einer Hydroxygruppe entspricht.
$R^3$ Wasserstoff, Halogen, $C_1$-$C_4$-Alkyl, $C_1$-$C_4$-Alkoxy oder ein Carbonsäureamid welches am Stickstoff durch eine Phenylgruppe, die gegebenenfalls substituiert sein,
$R^4$ Wasserstoff, Halogen oder eine Sulfonsäuregruppe,
$R^5$ Wasserstoff, Halogen, $C_1$-$C_4$-Alkyl, $C_1$-$C_4$-Alkoxy oder eine Sulfonsäuregruppe,
$R^6$,$R^7$ Wasserstoff, Halogen. $C_1$-$C_4$-Alkyl, $C_1$-$C_4$-Alkoxy,
$R^8$ Wasserstoff, Halogen, $C_1$-$C_4$-Alkyl, $C_1$-$C_4$-Alkoxy oder Acetamido,
$R^9$ Hydroxy oder eine Bindung zur Azogruppe bedeutet,
$R^{10}$ Hydroxy bzw. eine Bindung zur Azogruppe bedeutet wenn $R^9$ einer Hydroxygruppe entspricht.
$R^{11}$ Wasserstoff, Halogen, Methoxy oder eine Carboxylgruppe,
$R^{12}$ Wasserstoff, Halogen, $C_1$-$C_4$-Alkoxy oder eine Sulfonsäuregruppe,
$R^{13}$ Wasserstoff, Halogen, $C_1$-$C_4$-Alkyl, $C_1$-$C_4$-Alkoxy oder eine Sulfonsäuregruppe,
$R^{14}$ Wasserstoff, Halogen, $C_1$-$C_4$-Alkyl, $C_1$-$C_4$-Alkoxy oder Nitro,
$R^{15}$ Wasserstoff, Halogen, $C_1$-$C_4$-Alkyl, $C_1$-$C_4$-Alkoxy oder eine Sulfonsäuregruppe,
$R^{16}$ Wasserstoff oder Halogen,
$R^{17}$ Wasserstoff, Halogen oder eine Sulfonsäuregruppe,
$R^{18}$ Wasserstoff, Halogen, Hydroxy oder eine Sulfonsäuregruppe,
$R^{19}$ Wasserstoff, Halogen, Methyl, Methoxy oder eine Sulfonsäuregruppe,
$R^{20}$ Wasserstoff, Halogen oder $C_1$-$C_4$-Alkyl und
$R^{21}$ eine Bindung zur Azogruppe bedeutet.

wobei $C_1$-$C_4$ dabei Methyl, Ethyl, Propyl, Butyl, Isopropyl, Isobutyl oder tert-Butyl bedeutet
sowie deren Tautomere, physiologisch verträglichen Salze und Prodrugs dieser Verbindungen zur Herstellung von Arzneimitteln zur Behandlung und Prävention von Thrombopenien und Anämien.

**2.** Bevorzugte Verbindungen der allgemeinen Formel **I**, gemäß Anspruch 1, wobei $R^1$ Hydroxy oder eine Bindung zur Azogruppe, $R^2$ Hydroxy bzw. eine Bindung zur Azogruppe bedeutet wenn $R^1$ einer Hydroxygruppe entspricht, $R^3$ Wasserstoff, $R^4$ und $R^5$ Wasserstoff oder eine Sulfonsäuregruppe, $R^6$,$R^7$ und $R^9$ Wasserstoff, $R^9$ Hydroxy oder eine Bindung zur Azogruppe bedeutet, $R^{10}$ Hydroxy bzw. eine Bindung zur Azogruppe bedeutet wenn $R^9$ einer Hydroxygruppe entspricht, $R^{11}$ Wasserstoff, $R^{12}$ Wasserstoff, $C_1$-$C_4$-Alkoxy oder eine Sulfonsäuregruppe, $R^{13}$ Wasserstoff oder eine Sulfonsäuregruppe, $R^{14}$ Wasserstoff oder eine Nitrogruppe, $R^{15}$ Wasserstoff oder eine Sulfonsäuregruppe und $R^{16}$ Wasserstoff, $R^{17}$ Wasserstoff oder eine Sulfonsäuregruppe, $R^{18}$ Wasserstoff oder eine Sulfonsäuregruppe, $R^{19}$ Wasserstoff, Halogen, Methyl oder eine Sulfonsäuregruppe, $R^{20}$ Wasserstoff und $R^{21}$ eine Bindung zur Azogruppe bedeutet, wobei $C_1$-$C_4$ dabei Methyl, Ethyl, Propyl, Butyl, Isopropyl, Isobutyl oder tert-Butyl entspricht.

**3.** Neue Verbindungen der allgemeinen Formel **I**, in denen R1 einer einer Hydroxygruppe entspricht und R18 gleichzeitig eine Trifluormethylgruppe bedeutet, wobei $R^2$, $R^3$, $R^4$, R5, $R^6$, $R^7$, $R^8$, $R^{17}$, $R^{19}$, $R^{20}$ und $R^{21}$ die unter Anspruch 1 spezifizierten Bedeutungen haben.

**4.** Verfahren zur Herstellung von Verbindungen gemäß Anspruch 3 basierend auf der Umsetzung von Diazoniumsalzen der allgemeinen Formel **II** mit Naphtholen der allgemeinen Formel **III.**

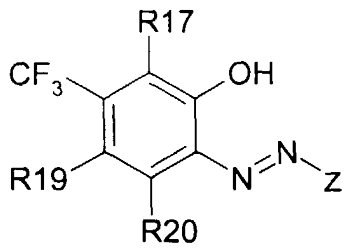

(II)

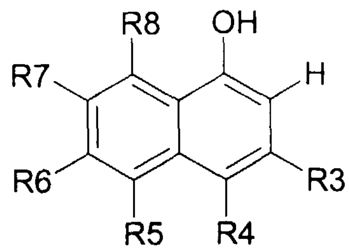

(III)

**5.** Verbindung 5-Hydroxy-6-(2-hydroxy-4-trifluormethyl-phenylazo)-naphthalin-1-sulfonsäure, ihrer E-, Z-Isomere, Tautomere, Salze und Ester.

**6.** Verwendung von Verbindungen der allgemeinen Formel **I**, gemäß Anspruch 1, 2 ,3 oder 5 zur Herstellung von Arzneimitteln zur Behandlung von Krankheiten, bei denen Thrombopoetin oder ein anderes Protein/Peptid, das an den mpl-Rezeptor bindet, als Therapeutikum eingesetzt wird.

**7.** Verwendung von Verbindungen gemäß Anspruch 1, 2, 3 oder 5 zur Herstellung eines Arzneimittels zur Stimulation der Plättchenbildung und Stammzellenmobilisation *in vivo.*

**8.** Verwendung von Verbindungen gemäß Anspruch 1, 2, 3 oder 5 zur Herstellung eines Arzneimittels zur *in vitro* Stimulation der Megakaryozyten- und Plättchenbildung.

**9.** Verwendung von Verbindungen gemäß Anspruch 1, 2, 3 oder 5 zur Herstellung eines Arzneimittels zur Stimulation der Erythrozytenbildung.

**10.** Verwendung von Verbindungen gemäß Anspruch 1, 2, 3 oder 5 zur Herstellung eines Arzneimittels zur Stammzellenexpansion.

Europäisches Patentamt

# EUROPÄISCHER RECHERCHENBERICHT

Nummer der Anmeldung
EP 99 12 2346

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | FR 1 171 642 A (COMPAGNIE FRANCAISE DES MATIERES COLORANTES) 28. Januar 1959 (1959-01-28) * das ganze Dokument * | 2 | A61K31/655 A61P7/04 A61P7/06 A61P43/00 C09B43/00 |
| X | US 2 987 513 A (CSHMIDT K-H. ET AL.) 6. Juni 1961 (1961-06-06) *Spalte 1-2 Verbindung (I) * | 2 | |
| X | CHEMICAL ABSTRACTS, vol. 128, no. 3, 19. Januar 1998 (1998-01-19) Columbus, Ohio, US; abstract no. 18441, MORRIS, ANGELA D. ET AL: "Eriochrome Black T, structurally related to suramin, inhibits angiogenesis and tumor growth in vivo" XP000912277 * Zusammenfassung * & ANTI-CANCER DRUGS (1997), 8(8), 746-755 , * Seite 747; Abbildung 1 * | 2 | |
| | | | RECHERCHIERTE SACHGEBIETE (Int.Cl.7) |
| | | | A61K C09B |
| X | GB 779 880 A (CIBA LIMITED) * Seite 1 - Seite 2 * | 2 | |
| A | | 3 | |
| A | GB 789 025 A (IMPERIAL CHEMICAL INDUSTRIES LIMITED) * das ganze Dokument * | 3 | |
| A | US 4 880 788 A (MOAKE ET AL.) 14. November 1989 (1989-11-14) * Spalte 4, Zeile 29 - Zeile 53 * * Spalte 10 * * Spalte 6, Zeile 18 - Zeile 68 * * Spalte 7 * | 1,2 | |

-/--

Der vorliegende Recherchenbericht wurde für alle Patentansprüche erstellt

| Recherchenort | Abschlußdatum der Recherche | Prüfer |
|---|---|---|
| DEN HAAG | 26. September 2000 | Gac, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 99 12 2346

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | BUNCE ET AL.: "Photorearragement of azoxybenzene to 2-Hydroxyazobenzene. Evidence for electrophilic substitution by oxygen" J. AM. CHEM. SOC., Bd. 99, Nr. 24, 1977, Seiten 7986-7991, XP000938768 * Seite 7988; Tabelle II * --- | 3,4 | |
| A,D | DE 177 925 C (ANILINFARBEN- & EXTRACT-FABRIKEN) * das ganze Dokument * --- | 4 | |
| A | CHEMICAL ABSTRACTS, vol. 82, no. 3, 20. Januar 1975 (1975-01-20) Columbus, Ohio, US; abstract no. 12031s, Seite 108; Spalte 12029; XP002148369 * Zusammenfassung * & LITMAN ET AL.: "Interaction of chemical carcinogens with plasma membranes. Effect of dimethylaminoazobenzene on erythrocyte osmotic fragility" BIOCHEM. BIOPHYS. RES. COMMUN., Bd. 60, Nr. 2, 1974, Seiten 865-871, * Zusammenfassung * --- | 1,9 | RECHERCHIERTE SACHGEBIETE (Int.Cl.7) |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 26. September 2000 | Gac, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 99 12 2346

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| T | CHEMICAL ABSTRACTS, vol. 132, no. 22, 29. Mai 2000 (2000-05-29) Columbus, Ohio, US; abstract no. 289849, WATANABE, MASAHITO ET AL: "Evaluation of micronucleus induction in rats by oil orange SS" XP002148370 * Zusammenfassung * | 1,9 | |
| A | & KANKYO HEN'IGEN KENKYU, 1999, 21, 251-253, --- | 1,9 | |
| A | CHEMICAL ABSTRACTS, vol. 113, no. 23, 3. Dezember 1990 (1990-12-03) Columbus, Ohio, US; abstract no. 206515, GEORGE, ELISABETH ET AL: "Effects of azobenzene and aniline in the rodent bone marrow micronucleus test" XP000912115 * Zusammenfassung * & CARCINOGENESIS (LONDON), 1990, 11, 1551-5, --- | 1,9 | RECHERCHIERTE SACHGEBIETE (Int.Cl.7) |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 26. September 2000 | Gac, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 99 12 2346

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| A | CHEMICAL ABSTRACTS, vol. 127, no. 12, 22. September 1997 (1997-09-22) Columbus, Ohio, US; abstract no. 159949, PEKINER, BILGEHAN ET AL: "In vitro effects of an azo compound on the hemolysis and unsaturated fatty acids of red blood cells" XP000912202 * Zusammenfassung * & CLIN. CHIM. ACTA, 1997, 263, 157-164, | 1,9 | |
| E | WO 00 35446 A (SMITH-KLEIN-BEECHAM) 22. Juni 2000 (2000-06-22) * das ganze Dokument, insbesondere Seiten 17 und 19 * | 1,2,6-8 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 26. September 2000 | Gac, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)